# EUROPEAN PATENT SPECIFICATION

(11) **EP 3 281 987 B1**
(45) Date of publication and mention of the grant of the patent: **16.03.2022**
(21) Application number: 17184830.2
(22) Date of filing: 04.08.2017
(51) Int. Cl.: C09B 67/08, C08F 2/44, C09B 67/02, G01N 33/53, G01N 33/58

(54) **CORE/SHELL-TYPE FLUORESCENT DYE-CONTAINING NANOPARTICLE AND PRODUCTION METHOD OF THE SAME**
FLUORESZIERENDE FARBSTOFFHALTIGE KERN-/HÜLLEN-TYP-NANOPARTIKEL UND HERSTELLUNGSVERFAHREN DAFÜR
DE TYPE COEUR/ÉCORCE NANOPARTICULE CONTENANT UN COLORANT FLUORESCENT ET SON PROCÉDÉ DE FABRICATION

(30) Priority: 09.08.2016 JP 2016156409
(43) Date of publication of application: 14.02.2018
(73) Proprietor: Konica Minolta, Inc., Tokyo 100-7015 (JP)
(72) Inventor: Ishii, Hironori, Tokyo, 100-7015 (JP); Aimiya, Takuji, Tokyo, 100-7015 (JP); Takanashi, Kensaku, Tokyo, 100-7015 (JP); Nakayama, Shin, Tokyo, 100-7015 (JP)
(74) Representative: Gille Hrabal Partnerschaftsgesellschaft mbB Patentanwälte

(56) References cited:
- EP-A1- 2 966 445
- EP-A1- 3 012 632
- Harald F. Krug: "Nanomaterial, Dokumentkennung RD-14-02336", Römpp Online, Version 3.26, 1 April 2009 (2009-04-01), XP055035458, Retrieved from the Internet: URL:http://www.roempp.com/prod/roempp.php [retrieved on 2012-08-14]

## Description

### Background

### Technological Field

The present invention relates to a core/shell-type fluorescent dye-containing nanoparticle and a method of producing the same. More particularly, the present invention relates to: a core/shell-type fluorescent dye-containing nanoparticle for immunohistochemical staining or live cell imaging, which has a high brightness and whose dye does not elute into physiological saline or buffer when used for immunohistochemical staining or live cell imaging; and a method of producing the same.

### Description of the Related Art

In immunohistochemical staining and live cell imaging, fluorescent dye-containing nanoparticles are used. Immunohistochemical staining is a technology of staining a protein or the like by allowing fluorescent dye-containing nanoparticles to bind to the protein or the like through utilization of immunoreaction. In live cell imaging, cultured cells are subjected to some kind of labeling and a specific substance is thereby visualized and, live cell imaging is a technology of indirectly visualizing a biomolecule by, for example, labeling the biomolecule with a fluorescent dye-containing nanoparticle and detecting its fluorescence signal. In such immunohistochemical staining and live cell imaging, it is necessary to inhibit the elution of fluorescent dye from fluorescent dye-containing nanoparticles bound to a protein, cell or the like into a dispersion medium.

As a means for inhibiting the elution of fluorescent dye into a dispersion medium, for example, Patent Document 1 discloses a core/shell-type particle obtained by synthesizing a thermosetting resin in the presence of a fluorescent dye, preparing a core particle composed of the thus synthesized fluorescent dye-containing thermosetting resin and then coating this core particle with a thermosetting resin. In immunohistochemical staining, even when this core/shell-type particle is subjected to an operation of exposure to physiological saline or the like, the fluorescent dye is unlikely to elute from the core/shell-type particle into a dispersion medium. The thermosetting resin used in the particle is, for example, a melamine resin, a polyurea, a polybenzoguanamine or a phenol resin. However, some fluorescent dyes are not easily encapsulated into a thermosetting resin, and the use of such a fluorescent dye that is not easily encapsulated into a thermosetting resin has a problem of yielding a low brightness. Therefore, the core/shell-type particle disclosed in Patent Document 1 has a limitation in that a dye easily encapsulated into a thermosetting resin must be selected and used therein.

Further, in the fields of fluorescent markers and fluorescent microparticles for ink-jet inks, as a technology for improving the light fastness of a fluorescent particle aqueous dispersion, Patent Document 2 discloses a core/shell-type particle in which a dye-containing particle obtained by impregnating a hydrophobic thermoplastic resin particle with a fluorescent dye is used as a core particle and this core particle is coated with a thermoplastic resin. When such a core/shell-type particle whose core and shell are both composed of a thermoplastic resin is exposed to an ionic liquid (e.g., physiological saline) or water, there is a problem that the dye elutes from the core/shell-type particle. Further, since the amount of a dye that can be incorporated into a particle through impregnation is small, the core/shell-type particle obtained in this manner cannot attain a high brightness and it is thus difficult to apply such a core/shell-type particle to immunohistochemical staining or live cell imaging.

Moreover, in the fields of fluorescent markers and fluorescent microparticles for ink-jet inks, as a technology for improving the light fastness of a fluorescent particle aqueous dispersion, Patent Document 3 discloses a core/shell-type particle in which a dye-containing particle obtained by impregnating a hydrophobic thermoplastic resin particle with a fluorescent dye is used as a core particle and this core particle is coated with an amino resin. This core/shell-type particle has a shell layer formed from a thermosetting resin and elution of the dye is thus inhibited even in physiological saline and water; however, since the dye is incorporated into the particle through impregnation, the particle has a low brightness as described above and it is thus difficult to apply the particle to immunohistochemical staining or live cell imaging.

### Related Art Documents

### Patent Documents

[Patent Document 1] JP 2015-108572 A
[Patent Document 2] JP 2002-338856 A
[Patent Document 3] JP 2004-189900 A

EP 3 012 632 A discloses fluorescent nanoparticles having a zeta potential of -10 mV to - 60 mV at pH 7.0 or a zeta potential of 0 mV to -10 mV in a buffer of pH 6.0 to 8.0. Moreover, it is disclosed that an appropriate electrical repulsive force can be generated between biomolecules that are generally negatively charged and the fluorescent nanoparticles. As a result, non-specific binding between the fluorescent nanoparticles and the biomolecules is surppressed and the fluorescent nanoparticles are specifically bound to a biomolecule to be stained through interaction stronger than the electrical repulsive force, so that the visibility of the specific biomolecule to be stained can be improved.

### Summary

### Problems to be Solved by the Invention

An object of the present invention is to provide: a fluorescent dye-containing nanoparticle which can be utilized in immunohistochemical staining and live cell imaging and has a high brightness and whose dye does not elutes into water, physiological saline, culture medium and the like; and a method of producing the same.

### Means for Solving the Problems

The present inventor solved the above-described problems by preparing core particles through polymerization of monomers for thermoplastic resin synthesis in the presence of a fluorescent dye and coating the resulting core particles each with a shell layer composed of a thermosetting resin. That is, the present invention relates to the following [1] to [12]:
[1] A method of producing core/shell-type fluorescent dye-containing nanoparticles for immunohistochemical staining or live cell imaging, the method comprising:
   the step 1 of polymerizing monomers for thermoplastic resin synthesis in the presence of a fluorescent dye and thereby preparing core particles composed of a thermoplastic resin comprising the fluorescent dye; and
   the step 2 of coating the core particles each with a shell layer composed of a thermosetting resin.
[2] The method of producing core/shell-type fluorescent dye-containing nanoparticles for immunohistochemical staining or live cell imaging according to [1], wherein the thermoplastic resin is a styrene resin, an acrylic resin, an acrylonitrile resin, an AS resin, an ASA resin, an alkyl methacrylate resin, a (poly)alkyl methacrylate resin, an acrylamide resin, or a resin formed by polymerization of a sulfonic acid group-containing monomer.
[3] The method of producing core/shell-type fluorescent dye-containing nanoparticles for immunohistochemical staining or live cell imaging according to [1] or [2], wherein the thermosetting resin is a melamine resin, a urea resin, an aniline resin, a guanamine resin, a phenol resin, a xylene resin, or a furan resin.
[4] The method of producing core/shell-type fluorescent dye-containing nanoparticles for immunohistochemical staining or live cell imaging according to any one of [1] to [3], wherein the fluorescent dye is at least one selected from rhodamine-based dye molecules, BODIPY-based dye molecules, squarylium-based dye molecules, cyanine-based dye molecules, oxazine-based dye molecules, carbopyronine-based dye molecules and aromatic dye molecules.
[5] The method of producing core/shell-type fluorescent dye-containing nanoparticles for immunohistochemical staining or live cell imaging according to any one of [1] to [4], wherein the step 1 is the step of emulsion-polymerizing the monomers for thermoplastic resin synthesis in the presence of the fluorescent dye and a surfactant.
[6] The method of producing core/shell-type fluorescent dye-containing nanoparticles for immunohistochemical staining or live cell imaging according to [5], wherein the step 1 is the step of performing the polymerization with an addition of a thermal polymerization initiator.
[7] The method of producing core/shell-type fluorescent dye-containing nanoparticles for immunohistochemical staining or live cell imaging according to [6], wherein the step 1 is the step of adding a polymerization initiator and subsequently allowing reaction to take place by vigorous stirring at 55 to 75°C for 4 to 24 hours, followed by vigorous staining at 80 to 90°C for 30 to 60 minutes.
[8] The method of producing core/shell-type fluorescent dye-containing nanoparticles for immunohistochemical staining or live cell imaging according to any one of [1] to [7], wherein the step 2 is the step of polymerizing monomers for thermosetting resin synthesis in the presence of the core particles.
[9] A core/shell-type fluorescent dye-containing nanoparticle for immunohistochemical staining or live cell imaging, the nanoparticle comprising:
   a core particle which is composed of a uniformly dispersed fluorescent dye-containing thermoplastic resin; and
   a shell layer which coats the core particle and is composed of a thermosetting resin.
[10] The core/shell-type fluorescent dye-containing nanoparticle for immunohistochemical staining or live cell imaging according to [9], wherein the thermoplastic resin is a styrene resin, an acrylic resin, an acrylonitrile resin, an AS resin, an ASA resin, an alkyl methacrylate resin, a (poly)alkyl methacrylate resin, an acrylamide resin, or a resin formed by polymerization of a sulfonic acid group-containing monomer.
[11] The core/shell-type fluorescent dye-containing nanoparticle for immunohistochemical staining or live cell imaging according to [9] or [10], wherein the thermosetting resin is a melamine resin, a urea resin, an aniline resin, a guanamine resin, a phenol resin, a xylene resin, or a furan resin.
[12] The core/shell-type fluorescent dye-containing nanoparticle for immunohistochemical staining or live cell imaging according to any one of [9] to [11], wherein the fluorescent dye is at least one selected from rhodamine-based dye molecules, BODIPY-based dye molecules, squarylium-based dye molecules, cyanine-based dye molecules, oxazine-based dye molecules, carbopyronine-based dye molecules and aromatic dye molecules.

### Effects of the Invention

By the method of producing fluorescent dye-containing nanoparticles according to the present invention, fluorescent dye-containing nanoparticles having a high brightness, whose dye does not elutes into water, physiological saline, culture medium and the like, can be produced, and the fluorescent dye-containing nanoparticles can be effectively utilized in immunohistochemical staining and live cell imaging.

Since the fluorescent dye-containing nanoparticles according to the present invention have a high brightness, they enable to quantify even a protein expressed at a low level in immunohistochemical staining. Further, since the fluorescent dye-containing nanoparticles according to the present invention contain a large amount of fluorescent dye, the nanoparticles have an improved light resistance and can thus endure a long-term exposure, making observation thereof under a fluorescence microscope easy. Moreover, even when the fluorescent dye-containing nanoparticles according to the present invention are exposed to physiological saline or buffer in a staining operation, the fluorescent dye does not elute from the nanoparticles.

Since the fluorescent dye-containing nanoparticles according to the present invention have a high brightness, the signal of a single particle can be detected even in live cell imaging. Furthermore, since the fluorescent dye-containing nanoparticles according to the present invention has high light resistance and their fluorescent dye does not elute into a culture medium, the nanoparticles can be easily observed over a long period of several hours to several days.

### Detailed Description of Embodiments

### Mode for Carrying Out the Invention

The method of producing core/shell-type fluorescent dye-containing nanoparticles for immunohistochemical staining or live cell imaging according to the present invention comprises: the step 1 of polymerizing monomers for thermoplastic resin synthesis in the presence of a fluorescent dye and thereby preparing core particles composed of a thermoplastic resin comprising the fluorescent dye; and the step 2 of coating the core particles each with a shell layer composed of a thermosetting resin.

The fluorescent dye-containing nanoparticles produced by the production method of the present invention are of a core/shell-type, each of which comprises a core particle occupying the central portion and a shell layer covering the core portion.

The method of producing fluorescent dye-containing nanoparticles according to the present invention is characterized in that core particles are prepared by polymerizing monomers for thermoplastic resin synthesis in the presence of a fluorescent dye and the core particles are each coated with a shell layer composed of a thermosetting resin.

When monomers are polymerized in the presence of a fluorescent dye, the resulting polymer grows while encapsulating the fluorescent dye therein. When core particles are prepared by synthesizing a thermosetting resin in the presence of a fluorescent dye as in conventional methods, since the thermosetting resin has a three-dimensional dense network structure, there is an advantage that the fluorescent dye encapsulated therein is strongly held by resin particles and is unlikely to be disengaged from the resin particles. On the other hand, however, there are a large number of fluorescent dyes having a low affinity for thermosetting resins and when such a fluorescent dye having a low affinity for thermosetting resins is used, the fluorescent dye is not likely to be encapsulated in the resulting thermosetting resin polymer upon the synthesis of a thermosetting resin, so that the thus prepared core particles contain only a small amount of the fluorescent dye. Core/shell-type fluorescent dye-containing nanoparticles obtained by coating such core particles with a shell layer have low brightness and cannot thus be effectively utilized in immunohistochemical staining or live cell imaging. Therefore, in conventional methods of preparing a core particle by synthesis of a thermosetting resin in the presence of a fluorescent dye, there is a limitation in that a fluorescent dye which has a high affinity for a thermosetting resin through interaction based on the electrical properties, hydrophobic properties and the like must be selected and used.

In contrast, in the method of producing fluorescent dye-containing nanoparticles according to the present invention where core particles are prepared by polymerizing monomers for thermoplastic resin synthesis in the presence of a fluorescent dye, since fluorescent dyes generally have higher affinity toward thermoplastic resins than toward thermosetting resins, the limitation of having to select and use a specific fluorescent dye is small and a wide range of fluorescent dyes can thus be used. Moreover, since thermoplastic resins have structures that are richer in flexibility than those of thermosetting resins, core particles containing a large amount of fluorescent dye can be prepared. On the other hand, since thermoplastic resins have looser structures than those of thermosetting resins, even after a fluorescent dye is once encapsulated into a thermoplastic resin and incorporated into the core particles, the fluorescent dye is easily disengaged from the core particles. Thus, in the method of producing fluorescent dye-containing nanoparticles according to the present invention, the core particles composed of a fluorescent dye-containing thermoplastic resin are each coated with a shell layer composed of a thermosetting resin. Since thermosetting resins have a three-dimensional dense network structure as described above, by coating the core particles with a shell layer composed of a thermosetting resin, even if the fluorescent dye is disengaged from the core particles, the disengaged fluorescent dye is blocked by the shell layer and thereby retained in the respective core/shell-type nanoparticles. Therefore, those core/shell-type nanoparticles that are produced by the method of producing fluorescent dye-containing nanoparticles according to the present invention can retain a large amount of fluorescent dye therein and thus have a high brightness.

As described above, according to the method of producing fluorescent dye-containing nanoparticles of the present invention, the limitation on fluorescent dye is small so that a wide range of fluorescent dyes can be utilized, and fluorescent dye-containing core/shell-type nanoparticles having a high brightness can be produced. Therefore, the fluorescent dye-containing core/shell-type nanoparticles produced by the production method of the present invention can be effectively utilized in immunohistochemical staining and live cell imaging.

The step 1 in the method of producing fluorescent dye-containing nanoparticles according to the present invention is the step of preparing core particles. In the step 1, monomers for thermoplastic resin synthesis are polymerized in the presence of a fluorescent dye. This results in the formation of core particles composed of a thermoplastic resin containing the fluorescent dye.

The thermoplastic resin is not particularly restricted and, for example, a styrene resin, an acrylic resin, an acrylonitrile resin, an AS resin, an ASA resin, an alkyl methacrylate resin, a (poly)alkyl methacrylate resin, an acrylamide resin, or a resin formed by polymerization of a sulfonic acid group-containing monomer can be suitably used. Among these resins, a styrene resin or an acrylonitrile resin is preferably used since it enables to more effectively inhibit the elution of the fluorescent dye and to thereby obtain fluorescent dye-containing core/shell-type nanoparticles having a high brightness.

As described above, since fluorescent dyes generally have high affinity for thermoplastic resins, a wide range of dyes can be used as the above-described fluorescent dye. Among fluorescent dyes, for example, at least one selected from rhodamine-based dye molecules (e.g., Texas Red-based dye molecules), BODIPY-based dye molecules, squarylium-based dye molecules, cyanine-based dye molecules, oxazine-based dye molecules, carbopyronine-based dye molecules and aromatic dye molecules (e.g., coumarin-based dye molecules) can be suitably used.

Specific examples of the rhodamine-based dye molecules include 5-carboxy-rhodamine, 6-carboxy-rhodamine, 5,6-dicarboxy-rhodamine, Rhodamine 6G, tetramethylrhodamine, X-rhodamine, Texas Red, Spectrum Red, LD700 PERCHLORATE, and Sulforhodamine 101.

Specific examples of the BODIPY-based dye molecules include BODIPY FL, BODIPY TMR, BODIPY 493/503, BODIPY 530/550, BODIPY 558/568, BODIPY 564/570, BODIPY 576/589, BODIPY 581/591, BODIPY 630/650, and BODIPY 650/665 (all of which are manufactured by Invitrogen).

Specific examples of the squarylium-based dye molecules include SRfluor, 680-carboxylate, 1,3-bis[4-(dimethylamino)-2-hydroxyphenyl]-2,4-dihydroxycyclobutenediylium dihydroxide, bis-1,3-bis[4-(dimethylamino)phenyl]-2,4-dihydroxycyclobutenediylium dihydroxide, bis-2-(4-(diethylamino)-2-hydroxyphenyl)-4-(4-(diethyliminio)-2-hydroxycyclohexa-2,5-dienylidene)-3-oxocyclobu t-1-enolate, 2-(4-(dibutylamino)-2-hydroxyphenyl)-4-(4-(dibutyliminio)-2-hydroxycyclohexa-2,5-dienylidene)-3-oxocyclobut-1-enolate, and 2-(8-hydroxy-1,1,7,7-tetramethyl-1,2,3,5,6,7-hexahydropyrido[3,2,1-*ij*]quinolin-9-yl)-4-(8-hydroxy-1,1,7,7-tetramet hyl-2,3,6,7-tetrahydro-1*H*-pyrido[3,2,1-*ij*]quinolinium-9(5*H*)-ylidene)-3-oxocyclobut-1-enolate.

Specific examples of the cyanine-based dye molecules include 1-butyl-2-[5-(1-butyl-1,3-dihydro-3,3-dimethyl-2*H*-indol-2-ylidene)-penta-1,3-dienyl]-3,3-dimethyl-3*H*-indolium hexafluorophosphate, 1-butyl-2-[5-(1-buty)-3,3-dimethyl-1,3-dihydro-indol-2-ylidene)-3-chloropenta-1,3-dienyl]-3,3-dimethyl-3*H*-indoliu m hexafluorophosphate, and 3-ethyl-2-[5-(3-ethyl-3*H-*benzothiazol-2-ylidene)-penta-1,3-dienyl]-benzothiazol-3-ium-iodide.

Specific examples of the oxazine-based dye molecules include Cresyl Violet, Oxazine 170, EVOblue 30, and Nile Blue.

Specific examples of the carbopyronine-based dye molecules include CARBOPYRONIN 149.

Specific examples of the coumarin-based dye molecules which are aromatic ring-containing dye molecules include coumarin 7, coumarin 30, Basic Yellow 40, 7-diethylamino-coumarin, 7-diethylamino-4-methyl-coumarin, 7-diethylamino-4-trifluoromethyl-coumarin, 7-(diethylamino)coumarin-3-carboxylic acid, ethyl 7-(diethylamino)coumarin-3-carboxylate, 7-diethylamino-3-(4-pyridinyl)-coumarin, 7-diethylamino-3-(2-thiophene)-coumarin, 7-diethylamino-4-carbonitrile-coumarin, 1,1,6,6,8-pentamethyl-2,3,5,6-tetrahydro-1*H*,4*H*-11-oxa-3a-aza-benzo[*de*]anthracen-10-one, 1,1,6,6-tetramethyl-8-trifluoro-2,3,5,6-tetrahydro-1*H*,4*H*-11-oxa-3a-aza-benzo[*de*]anthracen-10-one, coumarin 504T, 7-diethylamino-3-carboxaldehyde-coumarin, 1,1,6,6-tetramethyl-10-oxo-2,3,5.6-tetrahydro-1*H*,4*H*,10*H*-11-oxa-3a-aza-benzo[*de*]anthracen-9-carbonitrile, 9-(1*H*-benzimidazol-2-yl)-1.1,6,6-tetramethyl-2,3,5,6-tetrahydro-1*H*,4*H*-11-oxa-3a-aza-benzo[*de*]anthracen-10-one, 3-diethylamino-7-imino-7*H*-[1]benzopyrano[3',2':3,4]pyrido[1,2-a]benzimidazol-6-carbonitrile, 10,11,14,15-tetrahydro-6-imino-9,9,15,15-tetramethyl-6*H*,9*H-*benzimidazo[1",2":1':2']pyrido[4'3':2,3][1]benzopyran o[6,7,8-*ij*]quinolizine-7-carbonitrile, coumarin 6, coumarin 153, coumarin 102, coumarin 343, coumarin 334, coumarin 545, coumarin 504T, coumarin 545T, and 7-(diethylamino)-3-phenylcoumarin.

Specific examples of the aromatic ring-containing dye molecules other than coumarin-based dye molecules include *N*,*N*-bis-(2,6-diisopropylphenyl)-1,6,7,12-(4-*tert*-butylphenoxy)-perylene-3,4,9,10-tetracarboxylic acid diimide, *N*,*N'*-bis(2,6-diisopropylphenyl)-1,6,7,12-tetraphenoxyperylene-3,4:9,10-tetracarboxydiimide, *N*,*N*-bis(2,6-diisopropylphenyl)perylene-3,4,9,10-bis(dicarbimide), 16-*N*,*N*-bis(2,6-dimethylphenyl)perylene-3,4,9,10-tetracarboxylic acid diimide, 4,4'-[(8,16-dihydro-8,16-dioxodibenzo[*a,j*]perylene-2,10-diyl)dioxy]dibutyric acid, 2,10-dihydroxy-dibenzo[*a,j*]perylene-8,16-dione,2,10-bis(3-aminopropoxy)dibenzo[*a,j*]perylene-8,16-dione, 3,3'-[(8,16-dihydro-8,16-dioxodibenzo[*a*,*j*]perylene-2,10-diyl)dioxyldipropylamine, 17-bis(octyloxy)anthra[9,1,2-*cde*-]benzo[*rst*]pentaphene-5-10-dione, octadecanoic acid, 5,10-dihydro-5,10-dioxoanthra[9,1,2-*cde*]benzo[*rst*]pentaphene-16,17-diyl ester, dihydroxydibenzanthrone, benzenesulfonic acid, 4,4',4",4"'-[[2,9-bis[2,6-bis(1-methylethyl)phenyl]-1,2,3,8,9,10-hexahydro-1,3,8,10-tetraoxoanthra[2,1,9-*d*e*f*-6,5,10-*d'e'f*]diisoquinoline-5,6,12,13-tetrayl]tetrakis(oxy)]tetrakis-, benzeneethanaminium, 4,4',4",4'"-[[2,9-bis[2,6-bis(1-methylethyl)phenyl]-1,2,3,8,9,10-hexahydro-1,3,8,10-tetraoxoanthra[2,1,9-def:6,5,10-*d*'*e*'*f*]diisoquinoline-5,6,12,13-tetrayl]tetrakis(oxy)]tetrakis[*N*,*N*,*N*-trimethyl-], spiropyran, azobenzene, spiroperimidine, diarylethene, and pyranine.

The above-described core particles are prepared by polymerizing monomers for thermoplastic resin synthesis in the presence of a dye. The polymerization of the monomers for thermoplastic resin synthesis is preferably emulsion polymerization with an addition of a surfactant. For example, the monomers for thermoplastic resin synthesis are added to an aqueous solution containing a fluorescent dye and a surfactant, and the resultant is vigorously stirred at a temperature of usually at 55 to 75°C, preferably 68 to 72°C, for a period of about 10 minutes, preferably 10 to 15 minutes. Subsequently, a polymerization initiator is added, and the resultant is allowed to react with vigorous stirring at a temperature of 55 to 75°C, preferably 68 to 72°C, for a period of 4 to 24 hours, preferably 4 to 5 hours. The temperature of the resulting solution is increased to 80 to 90°C, preferably 80 to 82°C, and the solution is further vigorously stirred for 30 to 60 minutes, preferably 30 to 40 minutes. This reaction solution is usually separated into aggregates and a particle dispersion which is a supernatant. This particle dispersion is recovered from the reaction solution. After centrifuging the particle dispersion and removing the resulting supernatant which is a dispersion medium, ultrapure water is added to the precipitates, and the precipitates are ultrasonically dispersed. These processes of centrifugation, addition of ultrapure water to the resulting precipitates and ultrasonic dispersion are further repeated for twice or so. As a result, an aqueous dispersion of core particles is obtained.

As the monomers for thermoplastic resin synthesis, such monomers that yield a desired thermoplastic resin through polymerization are selected and used as appropriate.

The fluorescent dye is added in an amount of usually 1 to 50 mg, preferably 4 to 20 mg, with respect to 1 g of the monomers for thermoplastic resin synthesis.

The surfactant is not particularly restricted, and any surfactant that is normally used for emulsion polymerization reaction can be used. As the surfactant, any of anionic, non-ionic and cationic surfactants can be used. Examples of the anionic surfactants include sodium dodecylbenzenesulfonate. Examples of the non-ionic surfactants include polyethylene glycols and polyoxyethylene alkyl ethers. Examples of the cationic surfactants include dodecyltrimethylammonium bromide. These surfactants may be used individually, or two or more thereof may be used in combination.

As a commercially available surfactant, for example, "EMULGEN" (registered trademark, manufactured by Kao Corporation) or "NEOPELEX" (registered trademark, manufactured by Kao Corporation) can be suitably used. The effective ingredient of EMULGEN is a polyoxyethylene alkyl ether, and that of NEOPELEX is sodium dodecylbenzenesulfonate.

The surfactant(s) is/are added in an amount of usually 1 to 3 mg, preferably 1 to 2 mg, with respect to 1 g of the monomers for thermoplastic resin synthesis.

Examples of the polymerization initiator include thermal polymerization initiators that generate radicals by heat, such as azo compounds and peroxides. Examples of a preferred azo compound include V-50 (2,2'-azobis(2-methylpropionamidine)dihydrochloride), and examples of a preferred peroxide include ammonium persulfate. The polymerization initiator may be a redox polymerization initiator.

The polymerization initiator is added in an amount of usually 0.1 to 1.5 mg, preferably 0.3 to 0.45 mg, with respect to 1 g of the monomers for thermoplastic resin synthesis.

The core particles prepared in the above-described manner have an average particle size of usually 20 to 300 nm, preferably 50 to 200 nm. When the average particle size of the core particles is larger than 300 nm, the stainability may be a problem, whereas when the average particle size of the core particles is smaller than 20 nm, the visibility may be a problem. The average particle size of the core particles is a value obtained by taking an electron micrograph and measuring the cross-sectional areas of the fluorescent dye-containing nanoparticles under a scanning electron microscope (SEM) and then calculating the diameter of a circle having the respective measured values as its area (area-equivalent circle diameter). The same method of measuring the average particle size is also applied to the below-described fluorescent dye-containing core/shell-type nanoparticles.

Since the core particles are prepared by polymerizing monomers for thermoplastic resin synthesis in the presence of a fluorescent dye, a large amount of the fluorescent dye can be incorporated therein in a uniformly dispersed state. Therefore, those fluorescent dye-containing core/shell-type nanoparticles that are produced using core particles prepared by the step 1 of the method of producing fluorescent dye-containing nanoparticles according to the present invention can have a high brightness. In contrast, in cases where particles are prepared by polymerizing monomers for thermoplastic resin synthesis and core particles are subsequently prepared by impregnating the thus obtained particles with a fluorescent dye, the dye is incorporated only in the surface part of the resulting core particles and the core particles contain only a small amount of the dye. Therefore, those fluorescent dye-containing core/shell-type nanoparticles that are produced using core particles prepared by such a method cannot have a high brightness.

The step 2 in the method of producing fluorescent dye-containing nanoparticles according to the present invention is the step of coating the above-described core particles with a shell layer composed of a thermosetting resin. By the step 2, core/shell-type fluorescent dye-containing nanoparticles each comprising a core particle and a core particle-coating shell layer composed of a thermosetting resin are formed.

The thermosetting resin is not particularly restricted as long as it is capable of forming the above-described shell layer, and preferred examples thereof include melamine resins, urea resins, aniline resins, guanamine resins, phenol resins, xylene resins and furan resins.

A method of coating the core particles with a shell layer composed of a thermosetting resin is not particularly restricted as long as the core particles can be coated with the thermosetting resin such that disengagement of the fluorescent dye from the core particles is inhibited; however, a method of polymerizing monomers for thermosetting resin synthesis in the presence of the core particles is convenient and thus preferably employed.

For example, a surfactant and monomers for thermosetting resin synthesis are added to an aqueous dispersion of core particles which contain the core particles at a concentration of 0.1 to 2 mg/mL, preferably 0.3 to 0.7 mg/mL, and the resulting mixture is vigorously stirred with heating at a temperature of usually at 70 to 80°C, preferably 75 to 78°C, for a period of about 10 minutes, preferably 10 to 15 minutes. Subsequently, an acid catalyst is added, and the resultant is continued to be stirred with heating at about 70°C, preferably 75 to 78°C, for another 50 minutes or so, preferably 45 to 50 minutes. Thereafter, the mixture is heated to about 90°C, preferably 85 to 90°C, and vigorously stirred with heating for about 20 minutes, preferably 15 to 20 minutes. This reaction solution is centrifuged, and the resulting supernatant is removed. Ultrapure water is added to the precipitates, and the precipitates are ultrasonically dispersed. These processes of centrifugation, addition of ultrapure water to the resulting precipitates and ultrasonic dispersion are further repeated for twice or so. As a result, an aqueous dispersion of core/shell-type fluorescent dye-containing nanoparticles is obtained. Using a scanning electron microscope, the core/shell-type fluorescent dye-containing nanoparticles can be confirmed to have larger particle sizes than the core particles.

As the monomers for thermosetting resin synthesis, such monomers that yield a desired thermosetting resin through polymerization are selected and used as appropriate.

Examples of the acid catalyst include dodecylbenzenesulfonic acid, sulfamic acid, formic acid, acetic acid, sulfuric acid, hydrochloric acid, nitric acid, and p-toluenesulfonic acid.

The acid catalyst is added in an amount of usually 30 to 80 mg, preferably 40 to 50 mg, with respect to 1 mg of the core particles.

The thickness of the shell layer is preferably 15 to 30 nm, more preferably 20 to 30 nm.

The core/shell-type fluorescent dye-containing nanoparticles prepared in the above-described manner have an average particle size of usually 40 to 500 nm, preferably 50 to 200 nm. When the average particle size of the core particles is larger than 500 nm, the stainability may be a problem, whereas when the average particle size of the core particles is smaller than 40 nm, the visibility may be a problem.

### Examples

### [Example 1]

### [Preparation of Core Particles]

To a 6-mL screw tube, 1,960 µL of ultrapure water, 9.6 µL of a 0.5 M aqueous EDTA solution, 300 µL of a 10-mg/mL aqueous Basic Yellow 40 solution, 300 µL of a 10-w/v% aqueous sodium dodecylbenzenesulfonate solution and 300 µL of 10-w/v% nonylphenyl poly(20)oxyethylene were added. To this mixture, as monomers for thermoplastic resin synthesis, 300 µL of styrene, 60 µL of polypropylene glycol monomethacrylate and 30 µL of a 50%-by-mass aqueous sodium 2-acrylamide-2-methylpropanesulfonate solution were added. A 10 mm-long stirring bar was placed in the screw tube, and the added materials were stirred on a hot stirrer at 62°C and 15,000 rpm for 10 minutes. To the resulting mixture, 50 µL of a 10-w/v% aqueous V-50 solution was added to initiate polymerization. The mixture was stirred at 62°C and 15,000 rpm for 4 hours. Then, the mixture was further stirred at 85°C and 15,000 rpm for 1 hour.

Aggregates were removed from the resulting reaction solution to recover a particle dispersion. This particle dispersion was centrifuged at 4°C and 15,000 rpm for 60 minutes, followed by removal of the resulting supernatant. To the thus obtained colored precipitates, 1 mL of ultrapure water was added, and the precipitates were ultrasonically dispersed. The centrifugation and the dispersion with ultrapure water were repeated twice, whereby an aqueous dispersion of core particles (1) composed of a fluorescent dye-containing thermoplastic resin was obtained. The thus obtained core particles (1) had a particle size of 100 nm.

### [Production of Fluorescent Dye-Containing Core/Shell-Type Nanoparticles]

To a 6-mL screw tube, 250 µL of the aqueous dispersion of core particles (1) containing the core particles (1) at a concentration of 50 mg/mL, 160 µL of a 5%-by-mass aqueous EMULGEN 430 (polyoxyethylene oleyl ether, manufactured by Kao Corporation) solution and 120 µL of a 50%-by-mass aqueous NIKALAC MX-035 (methylated melamine resin, manufactured by Nippon Carbide Industries Co., Inc.) solution were added. A 10 mm-long stirring bar was placed in the screw tube, and the added materials were stirred on a hot stirrer at 70°C and 15,000 rpm for 15 minutes. As an acid catalyst, 100 µL of a 5%-by-mass aqueous dodecylbenzenesulfonic acid solution was further added. The resultant was stirred at 70°C and 15,000 rpm for 60 minutes and then at 90°C and 15,000 rpm for 30 minutes. This dispersion was centrifuged at 4°C and 15,000 rpm for 20 minutes, followed by removal of the resulting supernatant. To the thus obtained colored precipitates, 1 mL of ultrapure water was added, and the precipitates were ultrasonically dispersed. The centrifugation and the dispersion with ultrapure water were repeated twice, whereby an aqueous dispersion of fluorescent dye-containing core/shell-type nanoparticles (1) was obtained. Using a scanning electron microscope, the particle size of the core/shell-type nanoparticles (1) was confirmed to be larger than that of the core particles (1). The core/shell-type nanoparticles (1) had a particle size of 115 nm. The thus obtained aqueous dispersion contained the core/shell-type nanoparticles (1) at a concentration of 16.4 mg/mL.

### (Evaluation of Brightness)

The aqueous dispersion obtained above was diluted with ultrapure water to a fluorescent dye-containing core/shell-type nanoparticle (1) concentration of 10 pmol/L. For this diluted aqueous dispersion, the fluorescence intensity was measured using a fluorescence spectrophotometer (F-7000, manufactured by Hitachi High-Technologies Corporation). Based on the thus obtained fluorescence intensity, the brightness of the fluorescent dye-containing core/shell-type nanoparticles (1) was evaluated. The result thereof is shown in Table 1.

### (Evaluation of Amount of Fluorescent Dye Eluted into Dispersion Medium)

The aqueous dispersion obtained above was diluted with phosphate-buffered physiological saline (PBS) to a fluorescent dye-containing core/shell-type nanoparticle (1) concentration of 0.1 nmol/L. The thus diluted aqueous dispersion was incubated at 37°C for 3 hours and subsequently centrifuged at 4°C and 15,000 rpm for 60 minutes, followed by removal of the resulting supernatant. The fluorescence intensity of the supernatant was measured using a fluorescence spectrophotometer (F-7000, manufactured by Hitachi High-Technologies Corporation) to determine the fluorescent dye concentration in the supernatant. Based on the thus determined fluorescent dye concentration in the supernatant, the amount of the fluorescent dye eluted from the fluorescent dye-containing core/shell-type nanoparticles (1) into the dispersion medium was evaluated. The result thereof is shown in Table 1.

### [Example 2]

### [Preparation of Core Particles]

An aqueous dispersion of core particles (2) was obtained in the same manner as in Example 1, except that 200 µL of styrene, 100 µL of acrylonitrile, 60 µL of hydroxypropyl monomethacrylic acid and 30 µL sodium 2-acrylamide-2-methylpropanesulfonate were used as the monomers for thermoplastic resin synthesis. The thus obtained core particles (2) had a particle size of 100 nm.

### [Production of Fluorescent Dye-Containing Core/Shell-Type Nanoparticles]

An aqueous dispersion of fluorescent dye-containing core/shell-type nanoparticles (2) was obtained in the same manner as in Example 1, except that the aqueous dispersion of core particles (2) containing the core particles (2) at a concentration of 50 mg/mL was used in place of the aqueous dispersion of core particles (1). Using a scanning electron microscope, the particle size of the core/shell-type nanoparticles (2) was confirmed to be larger than that of the core particles (2). The core/shell-type nanoparticles (2) had a particle size of 115 nm. The thus obtained aqueous dispersion contained the core/shell-type nanoparticles (2) at a concentration of 20 mg/mL.

### (Evaluation of Brightness)

The brightness of the fluorescent dye-containing core/shell-type nanoparticles (2) was evaluated in the same manner as in Example 1. The result thereof is shown in Table 1.

### (Evaluation of Amount of Fluorescent Dye Eluted into Dispersion Medium)

The amount of the fluorescent dye eluted from the fluorescent dye-containing core/shell-type nanoparticles (2) into the dispersion medium was evaluated in the same manner as in Example 1. The result thereof is shown in Table 1.

### [Example 3]

### [Preparation of Core Particles]

An aqueous dispersion of core particles (3) was obtained in the same manner as in Example 1, except that 200 µL of styrene, 100 µL of acrylonitrile, 60 µL of polypropylene glycol monomethacrylic acid and 30 µL sodium 2-acrylamide-2-methylpropanesulfonate were used as the monomers for thermoplastic resin synthesis. The thus obtained core particles (3) had a particle size of 100 nm.

### [Production of Fluorescent Dye-Containing Core/Shell-Type Nanoparticles]

An aqueous dispersion of fluorescent dye-containing core/shell-type nanoparticles (3) was obtained in the same manner as in Example 1, except that the aqueous dispersion of core particles (3) containing the core particles (3) at a concentration of 50 mg/mL was used in place of the aqueous dispersion of core particles (1). Using a scanning electron microscope, the particle size of the core/shell-type nanoparticles (3) was confirmed to be larger than that of the core particles (3). The core/shell-type nanoparticles (3) had a particle size of 115 nm. The thus obtained aqueous dispersion contained the core/shell-type nanoparticles (3) at a concentration of 18 mg/mL.

### (Evaluation of Brightness)

The brightness of the fluorescent dye-containing core/shell-type nanoparticles (3) was evaluated in the same manner as in Example 1. The result thereof is shown in Table 1.

### (Evaluation of Amount of Fluorescent Dye Eluted into Dispersion Medium)

The amount of the fluorescent dye eluted from the fluorescent dye-containing core/shell-type nanoparticles (3) into the dispersion medium was evaluated in the same manner as in Example 1. The result thereof is shown in Table 1,

### [Example 4]

### [Preparation of Core Particles]

An aqueous dispersion of core particles (3) was obtained in the same manner as in Example 3. The thus obtained core particles (3) had a particle size of 100 nm.

### [Production of Fluorescent Dye-Containing Core/Shell-Type Nanoparticles]

An aqueous dispersion of fluorescent dye-containing core/shell-type nanoparticles (4) was obtained in the same manner as in Example 3, except that 100 µL. of 50%-by-mass urea (manufactured by Tokyo Chemical Industry Co., Ltd.) and 150 µL of 10%-by-mass formalin (manufactured by Tokyo Chemical Industry Co., Ltd.) were used in place of 120 µL of a 50%-by-mass aqueous NIKALAC MX-035 (methylated melamine resin, manufactured by Nippon Carbide Industries Co., Inc.) solution and 100 µL of 5%-by-mass dodecylbenzenesulfonic acid, respectively. Using a scanning electron microscope, the particle size of the core/shell-type nanoparticles (4) was confirmed to be larger than that of the core particles (3). The core/shell-type nanoparticles (4) had a particle size of 115 nm. The thus obtained aqueous dispersion contained the core/shell-type nanoparticles (4) at a concentration of 15.4 mg/mL.

### (Evaluation of Brightness)

The brightness of the fluorescent dye-containing core/shell-type nanoparticles (4) was evaluated in the same manner as in Example 1. The result thereof is shown in Table 1.

### (Evaluation of Amount of Fluorescent Dye Eluted into Dispersion Medium)

The amount of the fluorescent dye eluted from the fluorescent dye-containing core/shell-type nanoparticles (4) into the dispersion medium was evaluated in the same manner as in Example 1. The result thereof is shown in Table 1.

### [Example 5]

### [Preparation of Core Particles]

An aqueous dispersion of core particles (3) was obtained in the same manner as in Example 3. The thus obtained core particles (3) had a particle size of 100 nm.

### [Production of Fluorescent Dye-Containing Core/Shell-Type Nanoparticles]

An aqueous dispersion of fluorescent dye-containing core/shell-type nanoparticles (5) was obtained in the same manner as in Example 3, except that 120 µL of a 50%-by-mass aqueous benzoguanamine solution (manufactured by Tokyo Chemical Industry Co., Ltd.) and 150 µL of 10%-by-mass formalin (manufactured by Tokyo Chemical Industry Co., Ltd.) were used in place of 120 µL of a 50%-by-mass aqueous NIKALAC MX-035 (methylated melamine resin, manufactured by Nippon Carbide Industries Co., Inc.) solution and 100 µL of 5%-by-mass dodecylbenzenesulfonic acid, respectively. Using a scanning electron microscope, the particle size of the core/shell-type nanoparticles (5) was confirmed to be larger than that of the core particles (3). The core/shell-type nanoparticles (5) had a particle size of 115 nm. The thus obtained aqueous dispersion contained the core/shell-type nanoparticles (5) at a concentration of 10.3 mg/mL.

### (Evaluation of Brightness)

The brightness of the fluorescent dye-containing core/shell-type nanoparticles (5) was evaluated in the same manner as in Example 1. The result thereof is shown in Table 1.

### (Evaluation of Amount of Fluorescent Dye Eluted into Dispersion Medium)

The amount of the fluorescent dye eluted from the fluorescent dye-containing core/shell-type nanoparticles (5) into the dispersion medium was evaluated in the same manner as in Example 1. The result thereof is shown in Table 1.

### [Example 6]

### [Preparation of Core Particles]

An aqueous dispersion of core particles (4) was obtained in the same manner as in Example 3, except that 300 µL of a 10-mg/mL aqueous Sulforhodamine 101 solution was used in place of 300 µL of a 10-mg/mL aqueous Basic Yellow 40 solution. The thus obtained core particles (4) had a particle size of 100 nm.

### [Production of Fluorescent Dye-Containing Core/Shell-Type Nanoparticles]

An aqueous dispersion of fluorescent dye-containing core/shell-type nanoparticles (6) was obtained in the same manner as in Example 1, except that the aqueous dispersion of core particles (4) containing the core particles (4) at a concentration of 50 mg/mL was used in place of the aqueous dispersion of core particles (1). Using a scanning electron microscope, the particle size of the core/shell-type nanoparticles (6) was confirmed to be larger than that of the core particles (4). The core/shell-type nanoparticles (6) had a particle size of 115 nm. The thus obtained aqueous dispersion contained the core/shell-type nanoparticles (6) at a concentration of 20 mg/mL.

### (Evaluation of Brightness)

The brightness of the fluorescent dye-containing core/shell-type nanoparticles (6) was evaluated in the same manner as in Example 1. The result thereof is shown in Table 1.

### (Evaluation of Amount of Fluorescent Dye Eluted into Dispersion Medium)

The amount of the fluorescent dye eluted from the fluorescent dye-containing core/shell-type nanoparticles (6) into the dispersion medium was evaluated in the same manner as in Example 1. The result thereof is shown in Table 1.

### [Example 7]

### [Preparation of Core Particles]

An aqueous dispersion of core particles (5) was obtained in the same manner as in Example 3, except that 300 µL of a 10-mg/mL aqueous coumarin 30 solution was used in place of 300 µL of a 10-mg/mL aqueous Basic Yellow 40 solution. The thus obtained core particles (5) had a particle size of 100 nm.

### [Production of Fluorescent Dye-Containing Core/Shell-Type Nanoparticles]

An aqueous dispersion of fluorescent dye-containing core/shell-type nanoparticles (7) was obtained in the same manner as in Example 1, except that the aqueous dispersion of core particles (5) containing the core particles (5) at a concentration of 50 mg/mL was used in place of the aqueous dispersion of core particles (1). Using a scanning electron microscope, the particle size of the core/shell-type nanoparticles (7) was confirmed to be larger than that of the core particles (5). The core/shell-type nanoparticles (7) had a particle size of 115 nm. The thus obtained aqueous dispersion contained the core/shell-type nanoparticles (7) at a concentration of 16.8 mg/mL.

### (Evaluation of Brightness)

The brightness of the fluorescent dye-containing core/shell-type nanoparticles (7) was evaluated in the same manner as in Example 1. The result thereof is shown in Table 1.

### (Evaluation of Amount of Fluorescent Dye Eluted into Dispersion Medium)

The amount of the fluorescent dye eluted from the fluorescent dye-containing core/shell-type nanoparticles (7) into the dispersion medium was evaluated in the same manner as in Example 1. The result thereof is shown in Table 1.

### [Example 8]

### [Preparation of Core Particles]

An aqueous dispersion of core particles (6) was obtained in the same manner as in Example 3, except that 300 µL of a 1 0-mg/mL aqueous coumarin 7 solution was used in place of 300 µL of a 10-mg/mL aqueous Basic Yellow 40 solution. The thus obtained core particles (6) had a particle size of 100 nm.

### [Production of Fluorescent Dye-Containing Core/Shell-Type Nanoparticles]

An aqueous dispersion of fluorescent dye-containing core/shell-type nanoparticles (8) was obtained in the same manner as in Example 1, except that the aqueous dispersion of core particles (6) containing the core particles (6) at a concentration of 50 mg/mL was used in place of the aqueous dispersion of core particles (1). Using a scanning electron microscope, the particle size of the core/shell-type nanoparticles (8) was confirmed to be larger than that of the core particles (6). The core/shell-type nanoparticles (8) had a particle size of 115 nm. The thus obtained aqueous dispersion contained the core/shell-type nanoparticles (8) at a concentration of 15.6 mg/mL.

### (Evaluation of Brightness)

The brightness of the fluorescent dye-containing core/shell-type nanoparticles (8) was evaluated in the same manner as in Example 1. The result thereof is shown in Table 1.

### (Evaluation of Amount of Fluorescent Dye Eluted into Dispersion Medium)

The amount of the fluorescent dye eluted from the fluorescent dye-containing core/shell-type nanoparticles (8) into the dispersion medium was evaluated in the same manner as in Example 1. The result thereof is shown in Table 1.

### [Comparative Example 1]

An aqueous dispersion of fluorescent dye-containing thermoplastic resin particles (1) was obtained in the same manner as in "Preparation of Core Particles" of Example 3. The thus obtained thermoplastic resin particles (1) had a particle size of 100 nm.

### (Evaluation of Brightness)

The brightness of the thermoplastic resin particles (1) was evaluated in the same manner as in Example 1. The result thereof is shown in Table 1.

### (Evaluation of Amount of Fluorescent Dye Eluted into Dispersion Medium)

The amount of the fluorescent dye eluted from the thermoplastic resin particles (1) into the dispersion medium was evaluated in the same manner as in Example 1. The result thereof is shown in Table 1.

### [Comparative Example 2]

Fluorescent dye-containing core/shell-type nanoparticles (9) were produced in the same manner as in Example 10 described in JP 2015-108572A, except that Basic Yellow 40 was used in place of Sulforhodamine 101

### (Evaluation of Brightness)

The brightness of the fluorescent dye-containing core/shell-type nanoparticles (9) was evaluated in the same manner as in Example 1. The result thereof is shown in Table 1.

### (Evaluation of Amount of Fluorescent Dye Eluted into Dispersion Medium)

The amount of the fluorescent dye eluted from the fluorescent dye-containing core/shell-type nanoparticles (9) into the dispersion medium was evaluated in the same manner as in Example 1. The result thereof is shown in Table 1.

### [Comparative Example 3]

Fluorescent dye-containing thennosetting resin particles (1) were produced in the same manner as in Preparation Example 1 described in JP 2015-108572A, except that Basic Yellow 40 was used in place of Sulforhodamine 101.

### (Evaluation of Brightness)

The brightness of the thermosetting resin particles (1) was evaluated in the same manner as in Example 1. The result thereof is shown in Table 1.

### (Evaluation of Amount of Fluorescent Dye Eluted into Dispersion Medium)

The amount of the fluorescent dye eluted from the thermosetting resin particles (1) into the dispersion medium was evaluated in the same manner as in Example 1. The result thereof is shown in Table 1.

### [Comparative Example 4]

### [Preparation of Core Particles]

An aqueous dispersion of core particles (3) was obtained in the same manner as in Example 3. The thus obtained core particles (3) had a particle size of 100 nm.

### [Production of Fluorescent Dye-Containing Core/Shell-Type Nanoparticles]

To a 6-mL screw tube, 1,960 µL of ultrapure water, 9.6 µL of a 0.5 M aqueous EDTA solution, 300 µL of a 10-w/v% aqueous sodium dodecylbenzenesulfonate solution, 300 µL of 10-w/v% nonylphenyl poly(20)oxyethylene and 50 µL of the core particles (3) were added. To this mixture, as a monomer for thermoplastic resin synthesis, 200 µL of styrene (manufactured by Tokyo Chemical Industry Co., Ltd.) was added. A 10 mm-long stirring bar was placed in the screw tube, and the added materials were stirred on a hot stirrer at 62°C and 15,000 rpm for 10 minutes. To the resulting mixture, 50 µL of a 10-w/v% aqueous V-50 solution was added to initiate polymerization. The mixture was stirred at 62°C and 15,000 rpm for 4 hours. Then, the mixture was further stirred at 85°C and 15,000 rpm for 1 hour.

Aggregates were removed from the resulting reaction solution to recover a particle dispersion. This particle dispersion was centrifuged at 4°C and 15,000 rpm for 60 minutes, followed by removal of the resulting supernatant. To the thus obtained colored precipitates, 1 mL of ultrapure water was added, and the precipitates were ultrasonically dispersed. The centrifugation and the dispersion with ultrapure water were repeated twice, whereby an aqueous dispersion of core/shell-type nanoparticles (10) was obtained. The thus obtained core/shell-type nanoparticles (10) had a particle size of 120 nm. The thus obtained aqueous dispersion contained the core/shell-type nanoparticles (10) at a concentration of 9.5 mg/mL.

### (Evaluation of Brightness)

The brightness of the fluorescent dye-containing core/shell-type nanoparticles (10) was evaluated in the same manner as in Example 1. The result thereof is shown in Table 1.

### (Evaluation of Amount of Fluorescent Dye Eluted into Dispersion Medium)

The amount of the fluorescent dye eluted from the fluorescent dye-containing core/shell-type nanoparticles (10) into the dispersion medium was evaluated in the same manner as in Example 1. The result thereof is shown in Table 1.

### [Comparative Example 5]

An aqueous dispersion of fluorescent dye-containing thermoplastic resin particles (2) was obtained in the same manner as in "Preparation of Core Particles" of Example 3, except that 300 µL of a 10-mg/mL aqueous Sulforhodamine 101 solution was used in place of 300 µL of a 10-mg/mL aqueous Basic Yellow 40 solution. The thus obtained thermoplastic resin particles (2) had a particle size of 100 nm.

### (Evaluation of Brightness)

The brightness of the thermoplastic resin particles (2) was evaluated in the same manner as in Example 1. The result thereof is shown in Table 1.

### (Evaluation of Amount of Fluorescent Dye Eluted into Dispersion Medium)

The amount of the fluorescent dye eluted from the thermoplastic resin particles (2) into the dispersion medium was evaluated in the same manner as in Example 1. The result thereof is shown in Table 1.

### [Comparative Example 6]

An aqueous dispersion of fluorescent dye-containing thermoplastic resin particles (3) was obtained in the same manner as in "Preparation of Core Particles" of Example 1, except that 300 µL of a 10-mg/mL aqueous coumarin 30 solution was used in place of 300 µL of a 10-mg/mL aqueous Basic Yellow 40 solution. The thus obtained thermoplastic resin particles (3) had a particle size of 100 nm.

### (Evaluation of Brightness)

The brightness of the thermoplastic resin particles (3) was evaluated in the same manner as in Example 1. The result thereof is shown in Table 1,

### (Evaluation of Amount of Fluorescent Dye Eluted into Dispersion Medium)

The amount of the fluorescent dye eluted from the thermoplastic resin particles (3) into the dispersion medium was evaluated in the same manner as in Example 1. The result thereof is shown in Table 1.

### [Comparative Example 7]

An aqueous dispersion of fluorescent dye-containing thermoplastic resin particles (4) was obtained in the same manner as in "Preparation of Core Particles" of Example 1, except that 300 µL of a 10-mg/mL aqueous coumarin 7 solution was used in place of 300 µL of a 10-mg/mL aqueous Basic Yellow 40 solution. The thus obtained thermoplastic resin particles (4) had a particle size of 100 nm.

### (Evaluation of Brightness)

The brightness of the thermoplastic resin particles (4) was evaluated in the same manner as in Example 1. The result thereof is shown in Table 1.

### (Evaluation of Amount of Fluorescent Dye Eluted into Dispersion Medium)

The amount of the fluorescent dye eluted from the thermoplastic resin particles (4) into the dispersion medium was evaluated in the same manner as in Example 1. The result thereof is shown in Table 1.

### [Comparative Example 8]

Fluorescent dye-containing core/shell-type nanoparticles (11) were produced in the same manner as in Example 10 described in JP 2015-108572A.

### (Evaluation of Brightness)

The brightness of the fluorescent dye-containing core/shell-type nanoparticles (11) was evaluated in the same manner as in Example 1. The result thereof is shown in Table 1.

### (Evaluation of Amount of Fluorescent Dye Eluted into Dispersion Medium)

The amount of the fluorescent dye eluted from the fluorescent dye-containing core/shell-type nanoparticles (11) into the dispersion medium was evaluated in the same manner as in Example 1. The result thereof is shown in Table 1.

### [Comparative Example 9]

Fluorescent dye-containing core/shell-type nanoparticles (12) were produced in the same manner as in Example 10 described in JP 2015-108572A, except that coumarin 30 was used in place of Sulforhodamine 101.

### (Evaluation of Brightness)

The brightness of the fluorescent dye-containing core/shell-type nanoparticles (12) was evaluated in the same manner as in Example 1. The result thereof is shown in Table 1.

### (Evaluation of Amount of Fluorescent Dye Eluted into Dispersion Medium)

The amount of the fluorescent dye eluted from the fluorescent dye-containing core/shell-type nanoparticles (12) into the dispersion medium was evaluated in the same manner as in Example 1. The result thereof is shown in Table 1.

### [Comparative Example 10]

Fluorescent dye-containing core/shell-type nanoparticles (13) were produced in the same manner as in Example 10 described in JP 2015-108572A, except that coumarin 7 was used in place of Sulforhodamine 101.

### (Evaluation of Brightness)

The brightness of the fluorescent dye-containing core/shell-type nanoparticles (13) was evaluated in the same manner as in Example 1. The result thereof is shown in Table 1.

### (Evaluation of Amount of Fluorescent Dye Eluted into Dispersion Medium)

The amount of the fluorescent dye eluted from the fluorescent dye-containing core/shell-type nanoparticles (13) into the dispersion medium was evaluated in the same manner as in Example 1. The result thereof is shown in Table 1.

### [Comparative Example 11]

Fluorescent dye-containing thermosetting resin particles (2) were produced in the same manner as in Preparation Example 1 described in JP 2015-108572A.

### (Evaluation of Brightness)

The brightness of the thermosetting resin particles (2) was evaluated in the same manner as in Example 1. The result thereof is shown in Table 1.

### (Evaluation of Amount of Fluorescent Dye Eluted into Dispersion Medium)

The amount of the fluorescent dye eluted from the thermosetting resin particles (2) into the dispersion medium was evaluated in the same manner as in Example 1. The result thereof is shown in Table 1.

### [Comparative Example 12]

Fluorescent dye-containing thermosetting resin particles (3) were produced in the same manner as in Preparation Example 1 described in JP 2015-108572A, except that coumarin 30 was used in place of Sulforhodamine 101.

### (Evaluation of Brightness)

The brightness of the thermosetting resin particles (3) was evaluated in the same manner as in Example 1. The result thereof is shown in Table 1.

### (Evaluation of Amount of Fluorescent Dye Eluted into Dispersion Medium)

The amount of the fluorescent dye eluted from the thermosetting resin particles (3) into the dispersion medium was evaluated in the same manner as in Example 1. The result thereof is shown in Table 1.

### [Comparative Example 13]

Fluorescent dye-containing thermosetting resin particles (4) were produced in the same manner as in Preparation Example 1 described in JP 2015-108572A, except that coumarin 7 was used in place of Sulforhodamine 101.

### (Evaluation of Brightness)

The brightness of the thermosetting resin particles (4) was evaluated in the same manner as in Example 1. The result thereof is shown in Table 1.

### (Evaluation of Amount of Fluorescent Dye Eluted into Dispersion Medium)

The amount of the fluorescent dye eluted from the thermosetting resin particles (4) into the dispersion medium was evaluated in the same manner as in Example 1. The result thereof is shown in Table 1.

### [Comparative Example 14]

### [Preparation of Core Particles]

An aqueous dispersion of core particles (7) was obtained in the same manner as in Example 3, except that 300 µL of a 10-mg/mL aqueous Sulforhodamine 101 solution was used in place of 300 µL of a 1 0-mg/mL aqueous Basic Yellow 40 solution. The thus obtained core particles (7) had a particle size of 100 nm.

### [Production of Fluorescent Dye-Containing Core/Shell-Type Nanoparticles]

An aqueous dispersion of fluorescent dye-containing core/shell-type nanoparticles (13) was obtained in the same manner as in Comparative Example 3, except that the core particles (7) were used in place of the core particles (3). The thus obtained aqueous dispersion contained the core/shell-type nanoparticles (13) at a concentration of 9.2 mg/mL.

### (Evaluation of Brightness)

The brightness of the fluorescent dye-containing core/shell-type nanoparticles (13) was evaluated in the same manner as in Example 1. The result thereof is shown in Table 1.

### (Evaluation of Amount of Fluorescent Dye Eluted into Dispersion Medium)

The amount of the fluorescent dye eluted from the fluorescent dye-containing core/shell-type nanoparticles (13) into the dispersion medium was evaluated in the same manner as in Example 1. The result thereof is shown in Table 1.

### [Comparative Example 15]

### [Preparation of Core Particles]

An aqueous dispersion of core particles (8) was obtained in the same manner as in Example 3, except that 300 µL of a 10-mg/mL aqueous coumarin 30 solution was used in place of 300 µL of a 10-mg/mL aqueous Basic Yellow 40 solution. The thus obtained core particles (8) had a particle size of 100 nm.

### [Production of Fluorescent Dye-Containing Core/Shell-Type Nanoparticles]

An aqueous dispersion of fluorescent dye-containing core/shell-type nanoparticles (14) was obtained in the same manner as in Comparative Example 3, except that the core particles (8) were used in place of the core particles (3). The thus obtained aqueous dispersion contained the core/shell-type nanoparticles (14) at a concentration of 9.1 mg/mL.

### (Evaluation of Brightness)

The brightness of the fluorescent dye-containing core/shell-type nanoparticles (14) was evaluated in the same manner as in Example 1. The result thereof is shown in Table 1.

### (Evaluation of Amount of Fluorescent Dye Eluted into Dispersion Medium)

The amount of the fluorescent dye eluted from the fluorescent dye-containing core/shell-type nanoparticles (14) into the dispersion medium was evaluated in the same manner as in Example 1. The result thereof is shown in Table 1.

### [Comparative Example 16]

### [Preparation of Core Particles]

An aqueous dispersion of core particles (9) was obtained in the same manner as in Example 3, except that 300 µL of a 10-mg/mL aqueous coumarin 7 solution was used in place of 300 µL of a 10-mg/mL aqueous Basic Yellow 40 solution. The thus obtained core particles (9) had a particle size of 100 nm.

### [Production of Fluorescent Dye-Containing Core/Shell-Type Nanoparticles]

An aqueous dispersion of fluorescent dye-containing core/shell-type nanoparticles (15) was obtained in the same manner as in Comparative Example 3, except that the core particles (9) were used in place of the core particles (3). The thus obtained aqueous dispersion contained the core/shell-type nanoparticles (15) at a concentration of 8.9 mg/mL.

### (Evaluation of Brightness)

The brightness of the fluorescent dye-containing core/shell-type nanoparticles (15) was evaluated in the same manner as in Example 1. The result thereof is shown in Table 1.

### (Evaluation of Amount of Fluorescent Dye Eluted into Dispersion Medium)

The amount of the fluorescent dye eluted from the fluorescent dye-containing core/shell-type nanoparticles (15) into the dispersion medium was evaluated in the same manner as in Example 1. The result thereof is shown in Table 1

**[Table 1]**

| | Resin constituting core particle⁽¹⁾ | Resin constituting shell layer | Fluorescent dye | Brightness | Elution of dye |
|---|---|---|---|---|---|
| | | | | Fluorescence intensity (%) | Dye concentration (µg/mL) |
| Example 1 | thermoplastic resin | thermosetting resin | Basic Yellow 40 | 70 | not detected |
| Example 2 | thermoplastic resin | thermosetting resin | Basic Yellow 40 | 85 | not detected |
| Example 3 | thermoplastic resin | thermosetting resin | Basic Yellow 40 | 100 | not detected |
| Example 4 | thermoplastic resin | thermosetting resin | Basic Yellow 40 | 98 | not detected |
| Example 5 | thermoplastic resin | thermosetting resin | Basic Yellow 40 | 98 | not detected |
| Example 6 | thermoplastic resin | thermosetting resin | Sulforhodamine 101 | 80 | not detected |
| Example 7 | thermoplastic resin | thermosetting resin | Coumarin 30 | 85 | not detected |
| Example 8 | thermoplastic resin | thermosetting resin | Coumarin 7 | 82 | not detected |
| Comparative Example 1 | thermoplastic resin | none | Basic Yellow 40 | 85 | 1.5 |
| Comparative Example 2 | thermosetting resin | thermosetting resin | Basic Yellow 40 | 72 | not detected |
| Comparative Example 3 | thermosetting resin | none | Basic Yellow 40 | 75 | 0.12 |
| Comparative Example 4 | thermoplastic resin | thermoplastic resin | Basic Yellow 40 | 84 | 1.3 |
| Comparative Example 5 | thermoplastic resin | none | Sulforhodamine 101 | 86 | 2.0 |
| Comparative Example 6 | thermoplastic resin | none | Coumarin 30 | 90 | 1.5 |
| Comparative Example 7 | thermoplastic resin | none | Coumarin 7 | 92 | 1.2 |
| Comparative Example 8 | thermosetting resin | thermoplastic resin | Sulforhodamine 101 | 72 | not detected |
| Comparative Example 9 | thermosetting resin | thermoplastic resin | Coumarin 30 | 65 | not detected |
| Comparative Example 10 | thermosetting resin | thermoplastic resin | Coumarin 7 | 67 | not detected |
| Comparative Example 11 | thermosetting resin | none | Sulforhodamine 101 | 95 | 0.15 |
| Comparative Example 12 | thermosetting resin | none | Coumarin 30 | 75 | 0.13 |
| Comparative Example 13 | thermosetting resin | none | Coumarin 7 | 77 | 0.15 |
| Comparative Example 14 | thermoplastic resin | thermoplastic resin | Sulforhodamine 101 | 90 | 1.8 |
| Comparative Example 15 | thermoplastic resin | thermoplastic resin | Coumarin 30 | 92 | 1.3 |
| Comparative Example 16 | thermoplastic resin | thermoplastic resin | Coumarin 7 | 90 | 1.2 |
| (1) Represents the whole particle in the absence of shell layer | | | | | |

## Claims

1. A method of producing core/shell-type fluorescent dye-containing nanoparticles having an average particle size of 40 to 500 nm for immunohistochemical staining or live cell imaging, said nanoparticle comprising:
a core particle which is composed of a uniformly dispersed fluorescent dye-containing thermoplastic resin; and
a shell layer which coats said core particle and is composed of a thermosetting resin;
wherein said method comprising:
the step 1 of polymerizing monomers for thermoplastic resin synthesis in the presence of a fluorescent dye and thereby preparing core particles composed of a thermoplastic resin comprising said fluorescent dye; and
the step 2 of coating said core particles each with a shell layer composed of a thermosetting resin.

2. The method of producing core/shell-type fluorescent dye-containing nanoparticles for immunohistochemical staining or live cell imaging according to claim 1, wherein said thermoplastic resin is a styrene resin, an acrylic resin, an acrylonitrile resin, an acrylonitrile-styrene resin, an acrylonitrile-styrene-acrylate resin, an alkyl methacrylate resin, a (poly)alkyl methacrylate resin, an acrylamide resin, or a resin formed by polymerization of a sulfonic acid group-containing monomer.

3. The method of producing core/shell-type fluorescent dye-containing nanoparticles for immunohistochemical staining or live cell imaging according to claim 1 or 2, wherein said thermosetting resin is a melamine resin, a urea resin, an aniline resin, a guanamine resin, a phenol resin, a xylene resin, or a furan resin.

4. The method of producing core/shell-type fluorescent dye-containing nanoparticles for immunohistochemical staining or live cell imaging according to any one of claims 1 to 3, wherein said fluorescent dye is at least one selected from rhodamine-based dye molecules, BODIPY-based dye molecules, squarylium-based dye molecules, cyanine-based dye molecules, oxazine-based dye molecules, carbopyronine-based dye molecules and aromatic dye molecules.

5. The method of producing core/shell-type fluorescent dye-containing nanoparticles for immunohistochemical staining or live cell imaging according to any one of claims 1 to 4, wherein said step 1 is the step of emulsion-polymerizing said monomers for thermoplastic resin synthesis in the presence of said fluorescent dye and a surfactant.

6. The method of producing core/shell-type fluorescent dye-containing nanoparticles for immunohistochemical staining or live cell imaging according to claim 5, wherein said step 1 is the step of performing said polymerization with an addition of a thermal polymerization initiator.

7. The method of producing core/shell-type fluorescent dye-containing nanoparticles for immunohistochemical staining or live cell imaging according to claim 6, wherein said step 1 is the step of adding a polymerization initiator and subsequently allowing reaction to take place by vigorous stirring at 55 to 75°C for 4 to 24 hours, followed by vigorous staining at 80 to 90°C for 30 to 60 minutes.

8. The method of producing core/shell-type fluorescent dye-containing nanoparticles for immunohistochemical staining or live cell imaging according to any one of claims 1 to 7, wherein said step 2 is the step of polymerizing monomers for thermosetting resin synthesis in the presence of said core particles.

9. A core/shell-type fluorescent dye-containing nanoparticle having an average particle size of 40 to 500 nm for immunohistochemical staining or live cell imaging, said nanoparticle comprising:
a core particle which is composed of a uniformly dispersed fluorescent dye-containing thermoplastic resin; and
a shell layer which coats said core particle and is composed of a thermosetting resin.

10. The core/shell-type fluorescent dye-containing nanoparticle for immunohistochemical staining or live cell imaging according to claim 9, wherein said thermoplastic resin is a styrene resin, an acrylic resin, an acrylonitrile resin, an acrylonitrile-styrene resin, an acrylonitrile-styrene-acrylate resin, an alkyl methacrylate resin, a (poly)alkyl methacrylate resin, an acrylamide resin, or a resin formed by polymerization of a sulfonic acid group-containing monomer.

11. The core/shell-type fluorescent dye-containing nanoparticle for immunohistochemical staining or live cell imaging according to claim 9 or 10, wherein said thermosetting resin is a melamine resin, a urea resin, an aniline resin, a guanamine resin, a phenol resin, a xylene resin, or a furan resin.

12. The core/shell-type fluorescent dye-containing nanoparticle for immunohistochemical staining or live cell imaging according to any one of claims 9 to 11, wherein said fluorescent dye is at least one selected from rhodamine-based dye molecules, BODIPY-based dye molecules, squarylium-based dye molecules, cyanine-based dye molecules, oxazine-based dye molecules, carbopyronine-based dye molecules and aromatic dye molecules.

## Patentansprüche

1. Ein Verfahren zur Herstellung von fluoreszierendem Farbstoff enthaltenden Nanopartikeln des Kern/Schale-Typs, welche eine durchschnittlichen Partikelgröße von 40 bis 500 nm aufweisen für die immunhistochemische Färbung oder die Abbildung lebender Zellen, diese Nanopartikel umfassen:
ein Kernpartikel, das aus einem gleichmäßig dispergierten, einen fluoreszierenden Farbstoff enthaltenden thermoplastischen Harz besteht; und
eine Hüllenschicht, die das Kernpartikel umhüllt und aus einem thermohärtenden Harz besteht;
wobei dieses Verfahren umfasst:
den Schritt 1 der Polymerisation von Monomeren für die Synthese eines thermoplastischen Harzes in Gegenwart eines fluoreszierenden Farbstoffs und dadurch die Herstellung von Kernpartikeln, die aus einem thermoplastischen Harz bestehen, das den fluoreszierenden Farbstoff enthält; und
den Schritt 2 des Beschichtens dieser Kernpartikel jeweils mit einer Hüllenschicht, die aus einem thermohärtenden Harz besteht.

2. Das Verfahren zur Herstellung von fluoreszierendem Farbstoff enthaltenden Nanopartikeln des Kem/Schale-Typs für die immunhistochemische Färbung oder die Abbildung lebender Zellen gemäß Anspruch 1, wobei dieses thermoplastische Harz ein Styrolharz, ein Acrylharz, ein Acrylnitrilharz ein Acrylnitril-Styrol-Harz, ein Acrylnitril-Styrol-Acrylat-Harz, ein Alkylmethacrylat-Harz, ein (Poly)alkylmethacrylat-Harz, ein Acrylamid-Harz oder ein Harz, gebildet durch Polymerisation eines Sulfonsäuregruppen enthaltenden Monomers ist.

3. Das Verfahren zur Herstellung von fluoreszierendem Farbstoff enthaltenden Nanopartikeln des Kem/Schale-Typs für die immunhistochemische Färbung oder die Abbildung lebender Zellen gemäß Anspruch 1 oder 2, wobei das thermohärtende Harz ein Melaminharz, ein Harnstoffharz, ein Anilinharz, ein Guanaminharz, ein Phenolharz, ein Xylolharz oder ein Furanharz ist.

4. Das Verfahren zur Herstellung von fluoreszierendem Farbstoff enthaltenden Nanopartikeln des Kem/Schale-Typs für die immunhistochemische Färbung oder die Abbildung lebender Zellen nach einem der Ansprüche 1 bis 3, wobei der fluoreszierende Farbstoff mindestens einer ist, der aus Farbstoffmolekülen auf Rhodaminbasis, Farbstoffmolekülen auf BODIPY-Basis, Farbstoffmolekülen auf Squaryliumbasis, Farbstoffmolekülen auf Cyaninbasis, Farbstoffmolekülen auf Oxazinbasis, Farbstoffmolekülen auf Carbopyroninbasis und aromatischen Farbstoffmolekülen ausgewählt ist.

5. Das Verfahren zur Herstellung von fluoreszierendem Farbstoff enthaltenden Nanopartikeln des Kem/Schale-Typs für die immunhistochemische Färbung oder die Abbildung lebender Zellen nach einem der Ansprüche 1 bis 4, wobei dieser Schritt 1 der Schritt der Emulsionspolymerisation dieser Monomere für die thermoplastische Harzsynthese in Gegenwart des fluoreszierenden Farbstoffs und eines Tensids ist.

6. Das Verfahren zur Herstellung von fluoreszierendem Farbstoff enthaltenden Nanopartikeln des Kem/Schale-Typs für die immunhistochemische Färbung oder die Abbildung lebender Zellen nach Anspruch 5, wobei dieser Schritt 1 der Schritt der Durchführung jener Polymerisation mit Zugabe eines thermischen Polymerisationsinitiators ist.

7. Das Verfahren zur Herstellung von fluoreszierendem Farbstoff enthaltenden Nanopartikeln des Kem/Schale-Typs für die immunhistochemische Färbung oder die Abbildung lebender Zellen nach Anspruch 6, wobei dieser Schritt 1 der Schritt der Zugabe eines Polymerisationsinitiators und des anschließenden Ermöglichens der Reaktion durch kräftiges Rühren bei 55 bis 75°C für 4 bis 24 Stunden, gefolgt von kräftigem Färben bei 80 bis 90°C für 30 bis 60 Minuten, ist.

8. Das Verfahren zur Herstellung von fluoreszierendem Farbstoff enthaltenden Nanopartikeln des Kem/Schale-Typs für die immunhistochemische Färbung oder die Abbildung lebender Zellen nach einem der Ansprüche 1 bis 7, wobei dieser Schritt 2 der Schritt der Polymerisation von Monomeren für die thermohärtende Harzsynthese in Gegenwart dieser Kernpartikel ist.

9. Einen fluoreszierenden Farbstoff enthaltenden Nanopartikel des Kern/Schale-Typs für die immunhistochemische Färbung oder die Abbildung lebender Zellen, dieser Nanopartikel umfasst:
ein Kernpartikel, das aus einem gleichmäßig dispergierten, einen fluoreszierenden Farbstoff enthaltenden thermoplastischen Harz besteht; und
eine Hüllenschicht, die das Kernpartikel umhüllt und aus einem thermohärtenden Harz besteht.

10. Der fluoreszierende Farbstoff enthaltende Nanopartikel des Kern/Schale-Typs für die immunhistochemische Färbung oder die Abbildung lebender Zellen nach Anspruch 9, wobei das thermoplastische Harz ein Styrolharz, ein Acrylharz, ein Acrylnitrilharz, ein Acrylnitril-Styrol-Harz, ein Acrylnitril-Styrol-Acrylatharz, ein Alkylmethacrylatharz, ein (Poly)alkylmethacrylatharz, ein Acrylamidharz oder ein Harz, gebildet durch Polymerisation eines Sulfonsäuregruppen enthaltenden Monomers ist.

11. Der fluoreszierende Farbstoff enthaltende Nanopartikel des Kern/Schale-Typs für die immunhistochemische Färbung oder die Abbildung lebender Zellen nach Anspruch 9 oder 10, wobei das thermohärtende Harz ein Melaminharz, ein Harnstoffharz, ein Anilinharz, ein Guanaminharz, ein Phenolharz, ein Xylolharz oder ein Furanharz ist.

12. Der fluoreszierende Farbstoff enthaltende Nanopartikel des Kern/Schale-Typs für die immunhistochemische Färbung oder die Abbildung lebender Zellen nach einem der Ansprüche 9 bis 11, wobei dieser fluoreszierende Farbstoff mindestens einer ist, der aus Farbstoffmolekülen auf Rhodaminbasis, Farbstoffmolekülen auf BODIPY-Basis, Farbstoffmolekülen auf Squaryliumbasis, Farbstoffmolekülen auf Cyaninbasis, Farbstoffmolekülen auf Oxazinbasis, Farbstoffmolekülen auf Carbopyroninbasis und aromatischen Farbstoffmolekülen ausgewählt ist.

## Revendications

1. Procédé de production de nanoparticules contenant un colorant fluorescent de type noyau/enveloppe ayant une taille de particule moyenne allant de 40 à 500 nm pour une coloration immunohistochimique ou une imagerie de cellules vivantes, ladite nanoparticule comprenant :
une particule de noyau qui est composée d'une résine thermoplastique contenant un colorant fluorescent uniformément dispersée ; et
une couche d'enveloppe qui recouvre ladite particule de noyau et est composée d'une résine thermodurcissable ;
dans lequel ledit procédé comprenant :
l'étape 1 de polymérisation de monomères pour la synthèse de résine thermoplastique en présence d'un colorant fluorescent et ainsi de préparation de particules de noyau composées d'une résine thermoplastique comprenant ledit colorant fluorescent ; et
l'étape 2 de revêtement de chacune desdites particules de noyau d'une couche d'enveloppe composée d'une résine thermodurcissable.

2. Procédé de production de nanoparticules contenant un colorant fluorescent de type noyau/enveloppe pour une coloration immunohistochimique ou une imagerie de cellules vivantes selon la revendication 1, dans lequel ladite résine thermoplastique est une résine styrène, une résine acrylique, une résine acrylonitrile, une résine acrylonitrile-styrène, une résine acrylonitrile-styrène-acrylate, une résine méthacrylate d'alkyle, une résine (poly)méthacrylate d'alkyle, une résine acrylamide ou une résine formée par polymérisation d'un monomère contenant un groupe acide sulfonique.

3. Procédé de production de nanoparticules contenant un colorant fluorescent de type noyau/enveloppe pour une coloration immunohistochimique ou une imagerie de cellules vivantes selon la revendication 1 ou 2, dans lequel ladite résine thermodurcissable est une résine mélamine, une résine urée, une résine aniline, une résine guanamine, une résine phénolique, une résine xylène ou une résine furannique.

4. Procédé de production de nanoparticules contenant un colorant fluorescent de type noyau/enveloppe pour une coloration immunohistochimique ou une imagerie de cellules vivantes selon l'une quelconque des revendications 1 à 3, dans lequel ledit colorant fluorescent est au moins un choisi parmi des molécules de colorant à base de rhodamine, des molécules de colorant à base de BODIPY, des molécules de colorant à base de squarylium, des molécules de colorant à base de cyanine, des molécules de colorant à base d'oxazine, des molécules de colorant à base de carbopyronine et des molécules de colorant aromatiques.

5. Procédé de production de nanoparticules contenant un colorant fluorescent de type noyau/enveloppe pour une coloration immunohistochimique ou une imagerie de cellules vivantes selon l'une quelconque des revendications 1 à 4, dans lequel ladite étape 1 est l'étape de polymérisation en émulsion desdits monomères pour la synthèse de résine thermoplastique en présence dudit colorant fluorescent et d'un tensioactif.

6. Procédé de production de nanoparticules contenant un colorant fluorescent de type noyau/enveloppe pour une coloration immunohistochimique ou une imagerie de cellules vivantes selon la revendication 5, dans lequel ladite étape 1 est l'étape de réalisation de ladite polymérisation avec l'ajout d'un initiateur de polymérisation thermique.

7. Procédé de production de nanoparticules contenant un colorant fluorescent de type noyau/enveloppe pour une coloration immunohistochimique ou une imagerie de cellules vivantes selon la revendication 6, dans lequel ladite étape 1 est l'étape consistant à ajouter un initiateur de polymérisation et ensuite à laisser la réaction avoir lieu par agitation vigoureuse à une température entre 55 et 75°C pendant 4 à 24 heures, suivie d'une coloration vigoureuse à une température entre 80 et 90°C pendant 30 à 60 minutes.

8. Procédé de production de nanoparticules contenant un colorant fluorescent de type noyau/enveloppe pour une coloration immunohistochimique ou une imagerie de cellules vivantes selon l'une quelconque des revendications 1 à 7, dans lequel ladite étape 2 est l'étape de polymérisation de monomères pour la synthèse de résine thermodurcissable en présence desdites particules de noyau.

9. Nanoparticule contenant un colorant fluorescent de type noyau/enveloppe ayant une taille de particule moyenne allant de 40 à 500 nm pour une coloration immunohistochimique ou une imagerie de cellules vivantes, ladite nanoparticule comprenant :
une particule de noyau qui est composée d'une résine thermoplastique contenant un colorant fluorescent uniformément dispersée ; et
une couche d'enveloppe qui recouvre ladite particule de noyau et est composée d'une résine thermodurcissable.

10. Nanoparticule contenant un colorant fluorescent de type noyau/enveloppe pour une coloration immunohistochimique ou une imagerie de cellules vivantes selon la revendication 9, dans laquelle ladite résine thermoplastique est une résine styrène, une résine acrylique, une résine acrylonitrile, une résine acrylonitrile-styrène, une résine acrylonitrile-styrène-acrylate, une résine méthacrylate d'alkyle, une résine (poly)méthacrylate d'alkyle, une résine acrylamide ou une résine formée par polymérisation d'un monomère contenant un groupe acide sulfonique.

11. Nanoparticule contenant un colorant fluorescent de type noyau/enveloppe pour une coloration immunohistochimique ou une imagerie de cellules vivantes selon la revendication 9 ou 10, dans laquelle ladite résine thermodurcissable est une résine mélamine, une résine urée, une résine aniline, une résine guanamine, une résine phénolique, une résine xylène ou une résine furannique.

12. Nanoparticule contenant un colorant fluorescent de type noyau/enveloppe pour une coloration immunohistochimique ou une imagerie de cellules vivantes selon l'une quelconque des revendications 9 à 11, dans laquelle ledit colorant fluorescent est au moins un choisi parmi des molécules de colorant à base de rhodamine, des molécules de colorant à base de BODIPY, des molécules de colorant à base de squarylium, des molécules de colorant à base de cyanine, des molécules de colorant à base d'oxazine, des molécules de colorant à base de carbopyronine et des molécules de colorant aromatiques.
